# EUROPEAN PATENT SPECIFICATION

(11) **EP 1 150 577 B1**
(45) Date of publication and mention of the grant of the patent: **18.10.2006**
(21) Application number: 00902498.5
(22) Date of filing: 14.01.2000
(51) Int. Cl.: A23L 1/0522, A61K 36/064, A61K 35/74, A61K 47/36, C12N 11/10

(54) **IMPROVED MICROBIAL PREPARATIONS**
VERBESSERTE MIKROBIELLE ZUBEREITUNGEN
PREPARATIONS MICROBIENNES AMELIOREES

(30) Priority: 14.01.1999 AU PP816899
(43) Date of publication of application: 07.11.2001
(73) Proprietor: Food Technology Innovations pty. Limited, NSW 2052 (AU)
(72) Inventor: CONWAY, Patricial, Lynne, La Perouse, NSW 2036 (AU); BROWN, Ian, Lewis, Gymea, NSW 2227 (AU); WANG, Xin, Chapel Hill, QLD 4069 (AU); LUCAS, Rachel, Jane, Maroubra, NSW 2035 (AU)
(74) Representative: Brown, Fraser Gregory James
(86) International application number: PCT/AU2000/000021
(87) International publication number: WO 2000/041576

(56) References cited:
- WO-A-96/08261
- WO-A-97/34591
- WO-A-97/34592
- WO-A-97/34615
- WO-A-99/04649
- BROWN ET. AL.: 'High amylose maize starch as a versatile prebiotic for use with probiotic bacteria.' FOOD AUSTRALIA vol. 50, no. 12, December 1998, pages 603 - 610, XP002905639

## Description

### Technical Field

The present invention relates generally to a process of preparing a microbial preparation containing microbes having increased survival/recovery rates in use. The microbial preparations obtainable by the process are particularly suitable for inclusion in prebiotic and probiotic preparations and products including food, feed, nutraceutical and pharmaceutical products containing probiotic microorganisms, and products fermented by added microorganisms.

### Background Art

Probiotic products, for example solutions, powders, tablets and capsules are orally administered for improving health. Similarly foods are consumed not only for sustenance but also for added health benefits such as through the addition of probiotic microorganisms. Animal feeds are also being prepared with added probiotic microorganisms in order to assist the growth and performance of animals. Recent trends of consuming probiotic compositions for health benefits has led to the use of probiotic microorganisms in a variety of preparations, as well as inclusion into a wide range of processed food and feed products, including processed milk-based products. Microorganisms are also added as starter cultures in order to produce a range of fermented foods e.g. milk, meat, vegetable products.

As used in this specification. a probiotic or probiotic microorganism is a live microbial feed supplement which beneficially effects the host animal by improving its intestinal microbial balance. This is the definition provided by R. Fuller (AFRC Institute of Food Research, Reading Laboratory, UK) in Journal of Applied Bacteriology, 1989. 66, pp. 365-378 "Probiotics in Man and Animals - A Review", and has subsequently been extended to include supplements and food for humans. A probiotic or probiotic microorganism also includes a live microbial supplement or pharmaceutical preparation which can be delivered to the nasal cavity or vaginal tract which beneficially affects the host animal by improving its microbial balance at the respective site of delivery.

The constitution and quantity of the gut microflora can be influenced by conditions or stress induced by disease, life style, travel, and other factors. If microorganisms which positively effect the health and well being of the individual can be encouraged to populate the large bowel, this should improve the physiological well being of the host.

The introduction of beneficial microorganisms, or probiotics, is normally accomplished by the ingestion of the microorganisms in foods, drinks, fermented dairy products such as yoghurts, capsules, confectionary and other forms in such a way that the organism arrives in a viable condition in the large bowel or other site of interest in the host.

One problem with the inclusion of probiotic microorganisms into processed food products is that the microorganisms often cannot survive in the food product for any length of time. During production and storage of the food products, there is often a substantial decrease in the numbers of viable microorganisms. For example, usual shelf-life of milk-based products is calculated on the period of time before spoilage of the product. When probiotic microorganisms are added to these products, the shelf-life stated for the product may not be applicable with regard to delivering the desired number of microorganisms to the gut to obtain the required beneficial effect.

Brown et al reported that if mice ingest beneficial microorganisms together with high amylose maize starch (a prebiotic), larger numbers of beneficial organisms can be found in their faeces; in "High amylose maize starch as a versatile prebiotic for use with probiotic bacteria" Food Australia, 50(12) 1998 p603 - 610. Their experiments indicate the starch granules act as a carrier to protect the microoganisms from the hostile conditions/stresses in the gastrointestinal tract.

Other uses of microbes include biocontrol and bioremediation. Microbial preparations including microbes suitable for these uses having increased growth/yield potential, or increased survival/recovery rates would be an advantage. For instance, certain bifidobacteria and acidophilus strains are active against *Escherichia coli* and *Salmonella spp* means that the bacteria have applications as biocontrol agents. Other microbes also have biocontrol applications - for instance, various fungi and *Bacillus* species. Furthermore, Pseudomonads are known to be efficacious for bioremediation and the present invention is applicable for promoting their survival. Another commonly used bioremediation microbe is Alcaligenes.

The present inventors have made the surprising discovery that the inclusion of resistant starch in the form of, or derived from, starches containing dietary fibre, in the growth medium for the microorganisms can increase growth and yield of the microorganisms, as well as increase the survival of microorganisms in microbial preparations or starter cultures and in food and feed products during production and over the shelf-life of these products, and improve rate of survival of the microbes during transit through the digestive tract.

Dietary fibre is defined as measured by the AOAC, International (Association of the Official Analytical Chemistry) Total dietary fiber in foods:
enzyme-gravimetric method (Method 985.29). Assoc. Off. Anal. Chemists, Official Methods of Analysis, 16th Ed. Arlington VA, USA, 1995. Further addition of the resistant starch to the microbial preparations after growth of the microbe also further enhances robustness of the microbes, thus leading to enhanced survival of the microorganisms. The discovery is also applicable to biocontrol and bioremediation preparations as well as food fermented by the addition of microorganisms, starter cultures, since more robust starter cultures can also be produced by growth on resistant starch medium and/or addition of resistant starch to subsequent products as described above.

### Disclosure of the Invention

According to a first aspect of the present invention there is provided a process of preparing a microbial preparation having an increased survival/recovery rate in a product, the process comprising growing or culturing microbes in media based on, or containing, resistant starch, and harvesting the cultured microbes, the harvested microbes having increased survival/recovery rate when subsequently incorporated in a product as compared with the same microbes grown or cultured in a media without resistant starch.

Preferably, the product is a food, feed, nutraceutical. pharmaceutical, biocontrol or bioremediation product.

One form of resistant starch particularly suitable for the present invention is starch containing resistant starch, particularly high amylose maize starches or materials derived from high amylose maize starches.

It has been found that growth in media based on, or containing, resistant starch seems to enhance the microbes ability to bind to additional resistant starch added to the product therefore enhancing the growth/yield potential, or increased survival/recovery rate of the microbes.

The microbes used in the process of the present invention or the microbes in the improved microbial preparation obtainable by a process of the present invention may be particularly resistant to aeration, sheer, freeze drying, freezing, drying including high, medium and low water activity, elevated temperatures, low temperatures, pressure and pressure fluctuations, low pH, high pH, bile acids, moisture, high or low osmolarity, high salt, or combinations thereof.

The microbes used in the process of the present invention or the microbial preparation obtainable by a process according to the present invention is particularly suitable for use in probiotics, starter cultures, biocontrol or bioremediation agents.

According to a second aspect of the present invention there is provided a microbial preparation containing microbes having an increased survival rate/recovery in product as compared with the same microbes grown or cultured in a media without resistant starch preparable by the process according to the first aspect of the present invention.

According to a third aspect of the present invention there is provided a product containing microbes having an increased survival late/recovery as compared with the same microbes grown or cultured in a media without resistant starch, the product including a microbial preparation according to the second aspect of the present invention.

Preferably, the product is a food, feed, nutraceutical, pharmaceutical biocontrol or bioremediation product

According to a fourth aspect of the present invention there is provided use of resistant starch in microbial culture media to produce microbes which when subsequently used in a product after being harvested from the media have an increased survival rate as compared with the same microbes grown or cultured in media without resistant starch.

One form of resistant starch particularly suitable for the present invention is starch containing resistant starch. Preferably, the starches have an amylose content of at least 40% (w/w). In a preferred form the starch is from maize having an amylose content of at least 70% (w/w), at least 80% (w/w) or at least 90% (w/w). The starch can also be chemically, physically, or enzymically treated or modified. Chemical modification can be by oxidation, cross-bonding, etherification, esterification, acidification, dextrinisation, or mixtures thereof.

Starches can also be treated to enhance the resistant starch content by a number of physical or chemical means. One preferred means is to heat-treat starch in the presence of moisture (heat-moisture treatment) which can be achieved by a number of processes including heating under negative, atmospheric or positive pressure under elevated moisture, or cycling techniques through different temperatures and pressures. Heating can be in the order of 100 to 180°C, preferably around 120 to 150°C and moisture levels of 10 to 80%, preferably 20 to 60%. Repeated autoclaving and rapid cooling can also be used to increase the resistant starch content of starches. It will be appreciated that these processes and conditions can be changed to achieve the desired increase in the level of resistant starch in the starch being treated.

Treatment can also be by solvent extraction to remove fats and/or minerals from the starch.

There are a variety of probiotic microorganisms which are suitable for use in this invention including yeasts such as *Saccharomyces,* and bacteria such as the genera *Bifidobacterium, Bacteroides, Clostridium, Fusobacterium, Propionibacterium, Streptococcus, Enterococcus, Lactococcus, Staphylococcus, Leuconostoc, Peptostreptococcus* and *Lactobacillus.* The invention is not, however, limited to these particular microorganisms.

Preferably, the starter cultures include, but not limited, to lactic acid bacteria including lactobacillus, lactococcus and streptococcus, leuconostoc, and yeasts.

Preferably, the microorganisms for use in biocontrol or bioremediation products include bifidobacteria, acidophilus, fungi, *Bacillus* species, pseudomonads and Alcaligenes. It will be appreciated, however, that other species of microorganisms would also be suitable candidates for use according to the present invention.

Such arrangements may also include microorganisms of different strains or species, including non-starch utilisers, to interact and demonstrate improved growth and/or activity in the large bowel, nasal tract or vaginal tract.

In a preferred embodiment of the first, second, third and fourth aspects of the present invention, the microbial preparations are starter cultures or probiotic preparations which can be liquid, frozen or dried. The preparations may also include food and feed products containing other microbial additives. These products include fluid-based or solid-based products. Fluid-based food products include milk-based products where the edible ingredient is one or more milk-based ingredients including whole milk, milk solids, milk fat, cream, non-fat dried milk, any other component or derivative from milk that can be used in milk-based products, water-based fluids, cereal and plant extracts such as soy-based beverages and additives. Solid-based food products include snack bars, breakfast cereals, bread, confectionary, extruded food products, muesli bars, buns, biscuits, feed pellets, coated food products, tablets, food additives, health supplements, and pharmaceutical preparations.

The food products according to the third aspect of the present invention include any food product that is suitable to contain and deliver probiotic microorganisms. Examples include, but not limited to, food stuffs, fruit beverages, water ices, confectionary, coatings or covertures, yoghurts, yoghurt drinks, unfermented drinks, flavoured milk drinks, modified milk drinks, ice-creams, and dairy desserts.

Standard methods employed by the art can be used to prepare the food, feed, nutraceutical or pharmaceutical products according to the fourth aspect of the present invention. The resistant starch may be added separately, in combination with one or more of the ingredients that form part of the food product. The resistant starch when added separately may interact positively and/or synergistically with other ingredients in the food, feed, nutraceutical or pharmaceutical products.

The increase in survival rate of the microbes in the product relates to an increase over the expected survival rate of the same microbe in a similar product that does not contain the resistant starch-grown microbes.

In a preferred form, the resistant starch is the Hi-maize™ and Culture Pro™ range of resistant starch products. The resistant starch can be used in growth media at a concentration of about 0.01 to 10% (w/w) and in subsequent additions during preparation of the microbial preparations and in liquid food, feed, nutraceutical or pharmaceutical products. Preferably the resistant starch is used at 0.1 to 5% (w/w) and more preferably at about 1% (w/w). The starch containing resistant starch and/or dietary fibre can be used in dry food, feed, nutraceutical or pharmaceutical products and microbial preparations at a concentration of about 0.1 to 90% (w/w) total product or preparation. Preferably, the starch is used at about 1 to 10% (w/w).

Resistant starch has been found to be particularly suitable in fluid-based foods at a concentration of 0.1 to 5% w/v, in solid-based foods at 0.1 to 15% (w/w), and in feed, nutraceutical or pharmaceutical products at 0.1 to 95% (w/w).

A further advantage of the use of resistant starch is that additional resistant starch can also be added at any stage during the processing of the food, feed, nutraceutical or pharmaceutical product. The properties of the resistant starch are not adversely effected by the processes involved in producing processed products. One distinct advantage is that there is no need to add the resistant starch in sterile form at the end of the process. The product can undergo pasteurisation or the like without the concern of adversely effecting the starches' properties.

As used in this specification, "resistant starch" includes those forms defined as RS1, RS2, RS3 and RS4 as defined in Brown, McNaught and Moloney (1995) Food Australia 47: 272-275. Either modified or unmodified resistant starches or mixtures thereof can be used in the present invention.

In WO 94/03049 and WO 94/14342, high amylose starches are disclosed which are resistant starches and include maize starch having an amylose content of 50% (w/w) or more, particularly 80% (w/w) or more, rice starch having an amylose content of 27% (w/w) or more, or a wheat starch having 35% (w/w) or more. Furthermore, particular granular size ranges of starches having an amylose content of 50% or more and enhanced resistant starch content, these starches including maize, barley, and legumes. This invention is not, however, limited to these forms of resistant starch. For example, other forms of resistant starch are derived from sources such as bananas and tubers such as potatoes and modified forms thereof.

Chemical modifications, such as oxidation, cross-bonding, etherification, esterification, acidification, dextrinisation and the like are well known in this art as being suitable chemical treatments. Similarly, other modifications can be induced physically, enzymically or by other means well known to those skilled in the art.

It may also be useful to modify the degree of enzyme susceptibility of the resistant starch by altering the conformation or structure of the starch. Examples include acid or enzyme thinning and cross bonding using difunctional reagents, heat/moisture treatment and thermal annealing. Modification of the starch may also be carried out by manipulation of the crystalline nature of the starch. Such modification methods are known to the art and starches produced by these methods would be suitable for the present invention.

Preferably the resistant starches are derived or obtained from corn (maize). It will be appreciated, however, that other sources of resistant starch could be used in the present invention. Examples include cereals like sorghum, wheat, barley, oats and rice, tubers like potatoes and tapioca, legumes such as peas, and others including starches derived from genetically modified plant species.

As used herein, Hi-maize™ and Culture Pro™ refers to products obtained from high amylose starch containing over 70% amylose obtained from Starch Australasia Limited. High amylose starches containing resistant starch suitable for the present invention are described in AU 660560.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

In order that the present invention may be more clearly understood, preferred forms will be described with reference to the following examples and drawings.

### Brief Description of Drawings

Figure 1 shows growth of *Bifidobacterium* strain D with and without starch.
Figure 2 shows growth of *Bifidobacterium* strain E with and without starch.
Figure 3 shows growth of *Bifidobacterium* strain C with and without starch.
Figure 4 shows the survival/recovery of starch-grown microbes in a probiotic-based drink evaluated for *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%).
Figure 5 shows the survival/recovery of starch-grown microbes in a yogurt type fermented milk drink evaluated for *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%).
Figure 6 shows the survival/recovery of starch-grown microbes in orange juice evaluated for *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%).
Figure 7 shows the survival/recovery of starch-grown microbes in probiotic-based drink with the inclusion of additional resistant starch evaluated for *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%).
Figure 8 shows the survival/recovery of starch-grown microbes in yogurt type fermented milk product with the inclusion of additional resistant starch evaluated for *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%).
Figure 9 shows the survival/recovery of starch-grown microbes in orange juice with the inclusion of additional resistant starch evaluated for *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%).
Figure 10 shows the survival/recovery of the strains exposed to a freeze-thaw cycle after growth in the presence of resistant starch. *Bifidobacterium* strain C that had been grown in the presence of glucose (0.5%), or Starch 1 (0.5%), or Starch 1 (0.25%) + glucose (0.25%).
Figure 11 shows the survival/recovery of the strains exposed to a freeze-thaw cycle after growth in the presence of resistant starch. *Bifidobacterium* strain C that had been grown in the presence of Starch 1 (0.25%) + glucose (0.25%) with addition of further Starch 1.
Figure 12 shows the survival/recovery of the strains exposed to a freeze-thaw cycle after growth in the presence of resistant starch. *Bifidobacterium* strain C that had been grown in the presence of Starch 2 (0.5%) with addition of further Starch 1.
Figure 13 shows the survival of *Bifidobacterium* strain C grown in the presence of glucose (0.5%), or Starch 1 (0.25%) + glucose (0.25%), or Starch 2 (0.5%), or Starch 2 (0.25%) + glucose (0.25%) and then placed in various types of yogurts without added starch
Figure 14 shows survival of *Bifidobacterium* strain C grown in the presence of glucose (0.5%), or Starch 1 (0.25%) + glucose (0.25%), or Starch 2 (0.5%), or Starch 2 (0.25%) + glucose (0.25%) and placed in various types of yogurts with added starch.

### Modes for Carrying Out the Invention

The present inventors have found that the inclusion of resistant starch to microbial growth media and optionally in subsequent stages of production of microbial preparations containing the resistant starch-grown microbes resulted in a surprising and unexpected increase in growth recovery and/or survival of the microbes during production and storage of the preparations and products.

### EXAMPLE 1

Bifidobacterium strain Lafti™ 13B was anaerobically grown on a basal agar medium (BM) supplemented with 1% (w/w) of either glucose or resistant starch (Culture Pro™). After growth, cells were harvested from the plates using phosphate buffered saline (PBS) and aliquots were mixed with either PBS or PBS containing the starch granules (10% w/w). Aliquots of the mixtures were freeze dried. The susceptibility of the Lafti™ 13B cells to low pH was evaluated by adding the bacterial mixtures before freeze drying and rehydrated after freeze drying, to glycine buffer at a pH of 3.5. The viable cells were enumerated by determining colony forming units using Tryptone Yeast Extract Peptone plates (TYP) when added to pH 3.5 and after 3 hours. The reduction of viability over the 3 hours is presented in Table 1. It was noted that cells grown in the presence of starch were more resistant and that the inclusion of the starch further enhanced the resistance.

**Table 1. The reduction of viability over the 3 hours of Bifidobacterium cells**

| | Presence of starch after growth | Reduction of viability |
|---|---|---|
| Glucose grown cells | - | 26 x 10² |
| Glucose grown cells | + | 15 x 10² |
| Starch grown cells | - | 26 |
| Starch grown cells | + | 5 |

### EXAMPLE 2

Bifidobacterium strain Lafti™ 13B was pre-cultured in Basal broth (BM) supplemented with 1% w/v glucose or high amylose maize starch granules (Culture Pro™). Anaerobically grown cultures were inoculated (10 µl) onto BM agar or into broth, both media containing 1% (w/v) glucose or Culture Pro™. Plates were either spot inoculated or spread and then incubated anaerobically for 48 hr. Growth in broth or cells harvested from spread plates were quantified by enumerating the colony forming units (CFU). Growth on spot-inoculated plates was quantified by measuring the size of the colony as well as the size of the cleared zone around the colony which was indicative of utilisation of the starch by the Bifidobacterium cells. It was noted that Lafti™ 13B grew more rapidly on starch-containing medium when pre-cultured using starch-containing medium and produced larger colonies and cleared zones on agar plates containing starch. Furthermore, the yield was greater from starch-containing media, compared to glucose media, for cells pre-cultured in both control broth (glucose) and starch broth.

### RESULTS

Recovery of viable microorganisms after growth in the presence of starch was higher and more rapid than glucose controls.

Growth in starch media enhanced the yield of microorganisms after exposure to stress conditions, for example, low pH, bile acids, acids, heat, moisture, pressure, freeze drying, spray drying, singly or in combination.

Pre-culture in starch medium prior to growth in starch medium enhanced recovery/survival after exposure to stress conditions as outlined above, as well as enhancing yield.

Growth in starch medium and then addition of starch enhanced resistance to stress conditions as outlined above.

The invention is further illustrated by the following examples using two starches containing resistant starch, referred to as Starch 1 (about 20% (w/w) resistant starch) and Starch 2 (about 60% (w/w) resistant starch) which were granular high amylose maize starches, as well as a number of *Bifidobacterium* strains, referred to as strains A, B, C, D, E and F.

### EXAMPLE 3

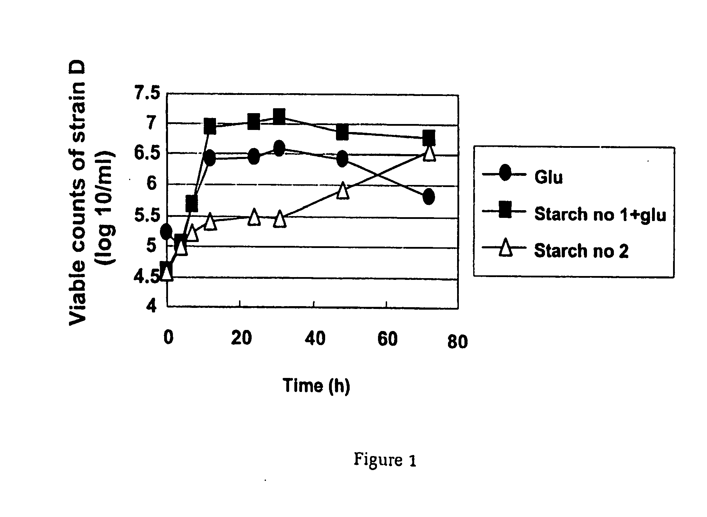

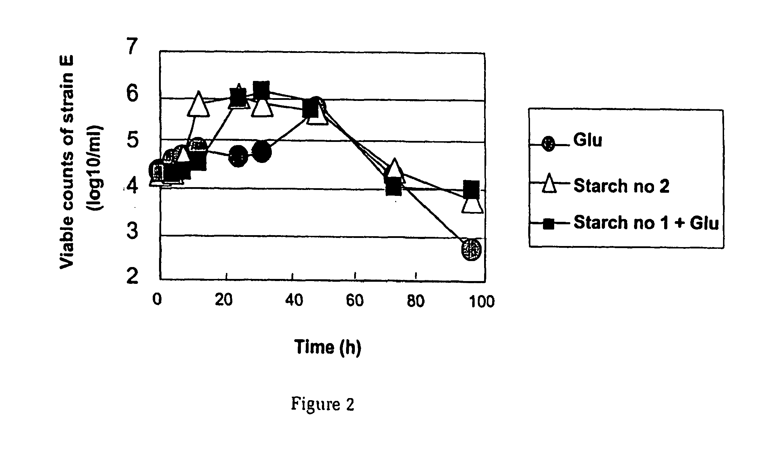

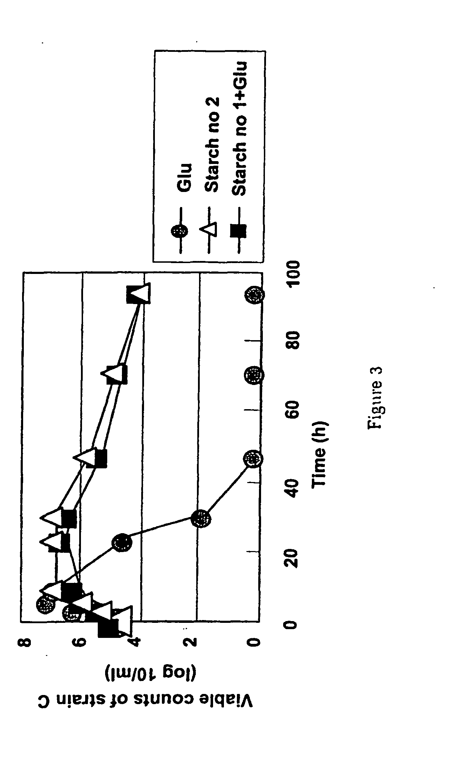

The growth and yield of *Bifidobacterium* strain D, *Bifidobacterium* strain E and *Bifidobacterium* strain C in the presence of natural granular maize starch was studied. A pre-culture of the strain was grown in 20 ml PYG broth for 18 hours and used to inoculate (0.1 ml aliquots) 20 ml of PY growth medium containing either glucose (0.5%), Starch 1 (0.5%), Starch 2 (0.5%), or a mixture of Starch 1 (0.25%) + glucose (0.25%). The cultures were incubated in an anaerobic chamber at 37°C and sampled at 0, 4, 7, 12, 24, 31, 48 and 72 hours to monitor the number of viable cells determined as colony forming units per ml of culture (CFU/ml⁻¹). The results presented in Figure 1 for *Bifidobacterium* strain D show that the inclusion of Starch 1 (0.25%) together with 0.25% glucose resulted in enhanced growth and yield of strain D compared to 0.5% glucose alone. For *Bifidobacterium* strain E (Figure 2) growth was more rapid in Starch 2 broth compared to the glucose control. Growth for strain E was also enhanced in Starch 1 + glucose broth compared to the glucose control. A different growth pattern and yield for *Bifidobacterium* strain C was noted. As shown in Figure 3, while the growth rate was not markedly different for the various broths, this strain when grown in glucose, died off rather rapidly once the maximum yield was obtained at about 18 hours. The strain maintained a higher yield when Starch 1 or Starch 2 were included.

### EXAMPLE 4

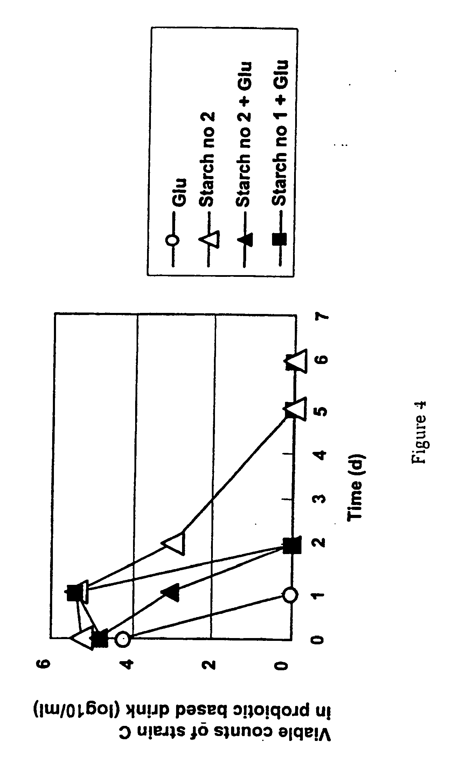

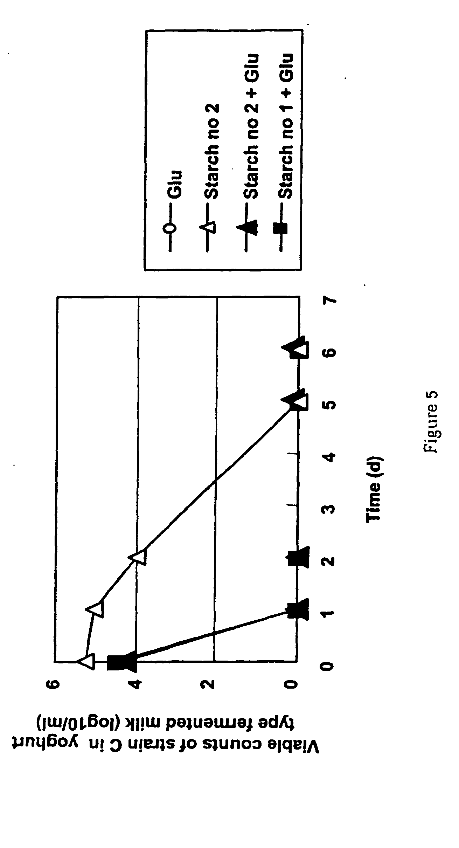

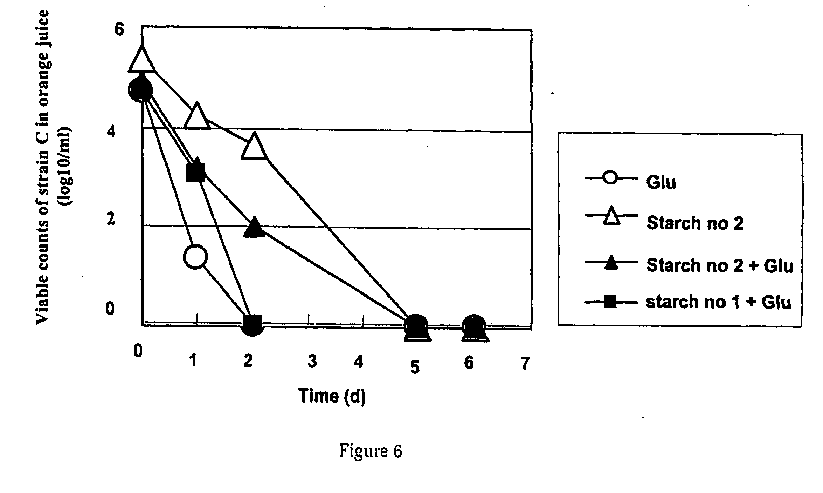

The survival/recovery of starch-grown microbes in foods was evaluated for *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%). The bifidobacterium cells were collected after 40 hours of anaerobic growth and diluted in the food product to yield approximately 10⁵ CFU per ml of food. The foods trialed included orange juice, a probiotic based drink and a yoghurt type fermented milk product. The food products containing the *Bifidobacterium* strain C cells were stored at room temperature (for accelerated storage conditions) and sampled at 0, 1, 2, 5 and 6 days to quantify the survival of *Bifidobacterium* strain C cells. From the results presented in Figure 4, enhanced survival of strain C in the probiotic based drink occurred when the strain was previously cultured in the presence of Starch 1 or 2. Similarly, enhanced survival of strain C was noted in the yoghurt type fermented milk product. For starch grown cells (Figure 5) and in orange juice for starch grown cells (Figure 6).

### EXAMPLE 5

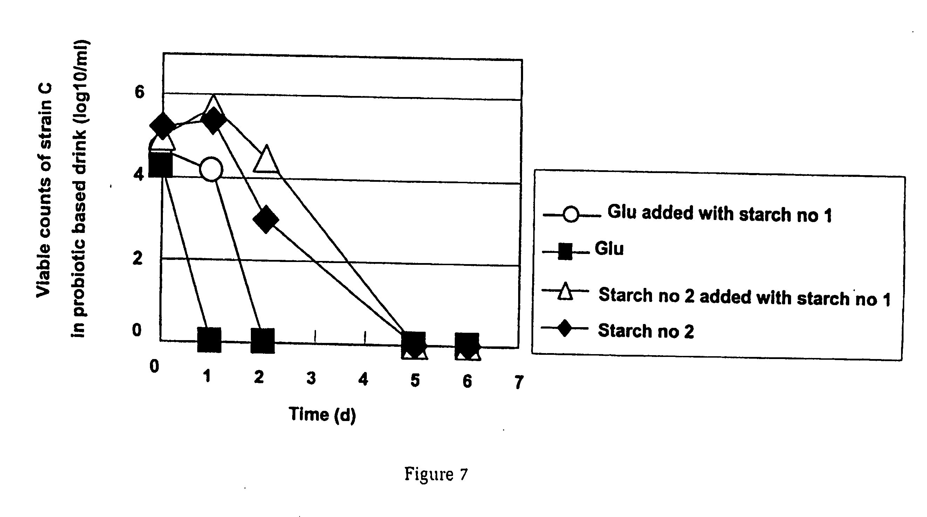

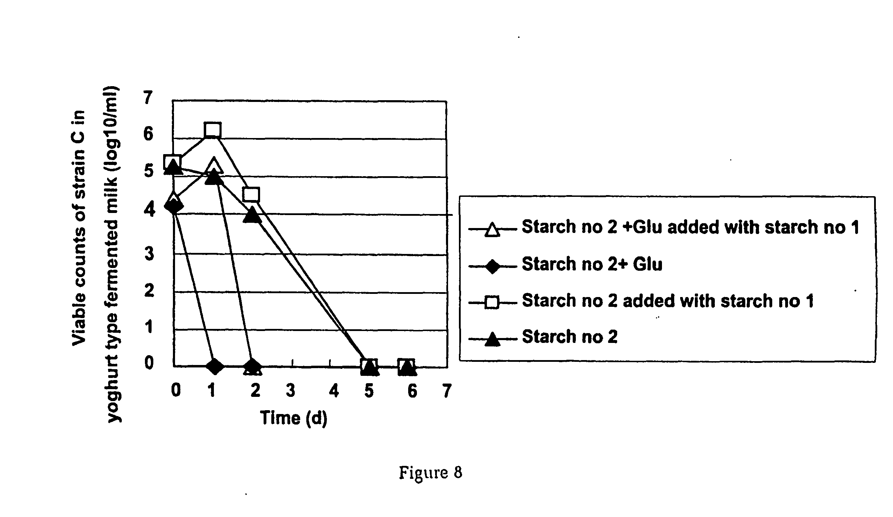

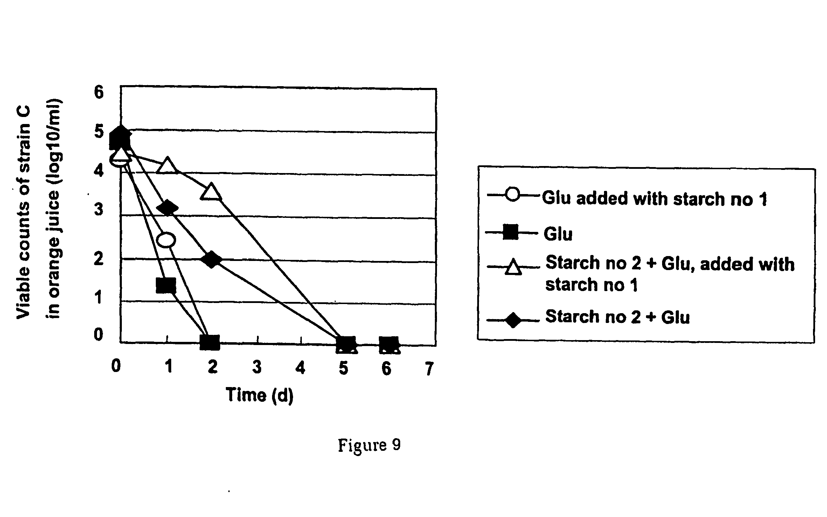

The inclusion of additional resistant starch to microorganisms cultured in the presence of resistant starch resulted in enhanced survival when the microorganisms were added to food products. This was investigated using *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%). The bifidobacterium cells were collected after 40 hours of anaerobic growth and mixed with 2.5% Starch 1 for 1 hour. The cells plus starch granules were then collected by centrifuging and added to the food product to yield approximately 10⁵ CFU per ml of food. The foods trialed included orange juice, a probiotic based drink and a yoghurt type fermented milk product. The food products containing the *Bifidobacterium* strain C cells with additional starch were stored at room temperature (for accelerated storage conditions) and sampled at 0, 1, 2, 5 and 6 days to quantify the survival of *Bifidobacterium* strain C cells. As presented in Figure 7, the inclusion of additional Starch 1 extended survival of the bacteria in a probiotic based drink irrespective what growth medium was used, and, as seen in Figure 8, in yoghurt type fermented milk product. The additional starch also extended survival of strain C in orange juice (Figure 9).

### EXAMPLE 6

Using *Bifidobacterium* strain C, 20 g female mice were orogastrically intubated with a suspension of cells harvested from glucose based plates or Starch 1 or Starch 2 based plates. The bacterial suspensions were standardised to a consistent optical density corresponding to approximately 10⁸ per ml of diluent. The bifidobacterium cells in freshly void faecal samples were enumerated using PAM agar plates and identification confirmed using PCR. Faecal samples were collected at 4, 10, 24 and 48 hours after administration of the bifidobacterium. No bifidobacterium cells were detected in mice dosed with glucose grown cells (Table 2). Mice dosed with strain C grown on Starch 1 agar had approximately log 7 CFU per gram wet weight in faeces detected 4 and 10 hours after oral administration. Numbers decreased in the 24 and 48 hours samples but were still detectable. When *Bifidobacterium* strain C grown on Starch 2 agar was orogastrically dosed to mice, approximately log 8 CFU per gram wet weight of faeces were detected 4 and 10 hours after administration, with a decrease for the 24 and 48 hour samples. It was concluded that growth on resistant starch based agar results in bifidobacterium cells which were more resistant to *in vivo* conditions which would include low stomach pH , bile acids and pancreatic enzymes.

**Table 2. Recovery of Bifidobacterium strain C from freshly void faeces of mice orogastrically dosed with Bifidobacterium strain C cells which had been cultured on either glucose agar, Starch 1 agar or Starch 2 agar. Results expressed as CFU per gram wet weight of faeces.**

| Time after dosage (h) | Glucose agar | Starch 1 agar | I Starch 2 agar |
|---|---|---|---|
| | (CFU per g wet weight) | | |
| 4 | ND | 5.8x10⁶ | 2.5 x 10⁸ |
| 10 | ND | 2.5x10⁷ | 6.0x10⁷ |
| 24 | ND | >10⁶ | > 10⁶ |
| 48 | ND | > 10⁵ | > 10⁵ |

| | | | |
|---|---|---|---|
| ND - none detected | | | |

### EXAMPLE 7

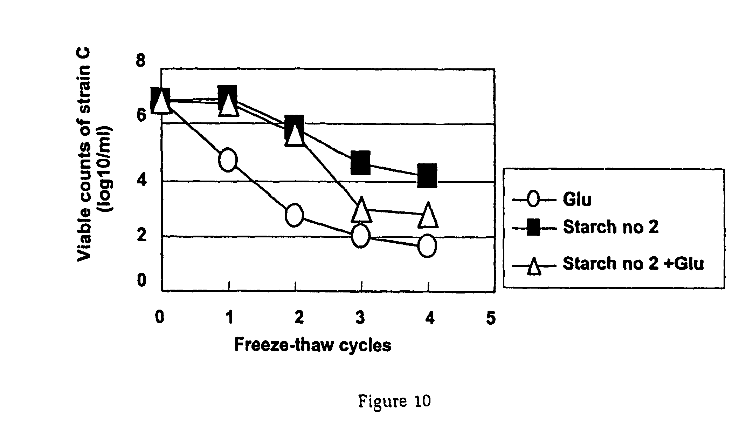

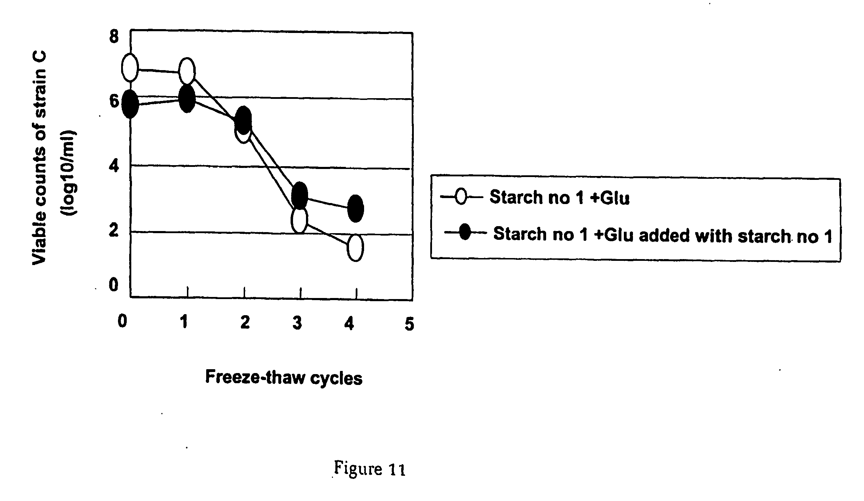

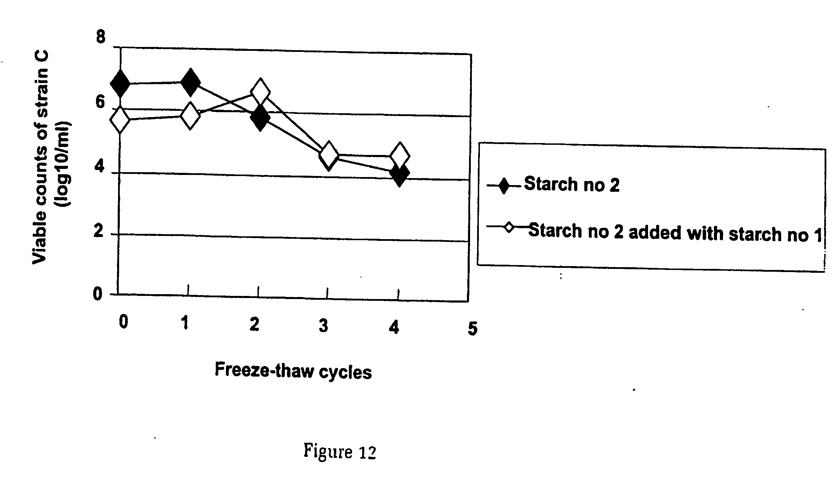

Microorganisms cultured in the presence of resistant starch were shown to be more resistant to physical stress as illustrated by the following example in which bifidobacterium strains were exposed to a freeze-thaw cycle after growth in the presence of resistant starch. *Bifidobacterium* strain C that had been grown in the presence of glucose (0.5%), or Starch 1 (0.25%) + glucose (0.25%), or Starch 2 (0.5%), or Starch 2 (0.25%) + glucose (0.25%). The bifidobacterium cells were collected after 40 hours of anaerobic growth and the samples ere divided into two. One portion was frozen directly (-20°C) and the other portion was mixed with 2.5% Starch 1 for 1 hour. The cells plus starch granules were then collected by centrifuging and also frozen at -20° C. The viable bifidobacterium cells were quantified after 0, 1, 2, 3, and 4 freeze thaw cycles which involved thawing the samples at room temperature each day for 4 days. As presented in Figure 10, growth in the presence of resistant starch prior to exposure to the physical stress enhanced survival of the bifidobacterium. Furthermore, the addition of more resistant starch before freezing improved further the survival of the Bifidobacterium cells (Figures 11 and 12).

### EXAMPLE 8

Bacteria grown in the presence of resistant starch and subsequently freeze dried prior to storage at elevated temperatures were more resistant to elevated temperatures than those grown in the absence of the starch. Three *Bifidobacterium* strains A, B and D were each grown in fermentors with pH control. The cell biomass was harvested and freeze dried. The dried powder was then stored at 42°C for 7 days and sampled daily for 4 days and then again on day 7 to quantify the viable bifidobacteria. The results are expressed as the colony forming units per gram of dried powder. As illustrated in Table 3, cells that were grown in the presence of Starch 1, survived better or were recovered at higher levels than those grown in the absence of the resistant starch.

**Table 3. Survival of freeze dried Bifidobacterium strains A, B and D at 42⁰C incubation**

| | Strain A | | Strain B | | Strain C | |
|---|---|---|---|---|---|---|
| Time (d) | No starch | With starch | No starch | With starch | No starch | With starch |
| 0 | 10.52 | 10.59 | 10.58 | 10.56 | 10.65 | 10.58 |
| 1 | 10.30 | 10.23 | 10.23 | 10.40 | 10.11 | 10.20 |
| 2 | 9.11 | 10.11 | 7.83 | 10.08 | 7.76 | 9.83 |
| 3 | 7.26 | 10.00 | 5.00 | 9.53 | 5.00 | 8.53 |
| 4 | 4.60 | 9.48 | 4.00 | 8.08 | 4.00 | 5.68 |
| 7 | 0.00 | 8.04 | 0.00 | 0.00 | 0.00 | 1.30 |

### EXAMPLE 9

The survival of *Clostridium butyricum* was monitored after growth in broth with no added starch. The cells were harvested and resuspended in pH 3.8 buffer containing 2.5% Starch 1 , 2.5% Starch 2, 2.5% cellulose or 2.5% starch 3 and the resultant viable cells quantified after 3 hours. The results are expressed as the percentage relative to the initial viable count. As can be seen in Table 4, the presence of Starch 2 enhanced survival. The cellulose did not protect against loss of viability as was noted for the resistant starch.

**Table 4. Survival of Clostridium butyricum grown in the absence of resistant starch and then combined with 2.5% Starch 1, 2 or 3 suspended in buffer of pH 3.8. The loss of viability was monitored after 3 hours exposure to the pH 3.8.**

| Starch addition | Percentage viable after 3 hours at pH 3.8 |
|---|---|
| No starch | 80.5 |
| Starch 1 | 71.3 |
| Starch 2 | 111.3 |
| Cellulose | ND |
| Starch 3 | 66.0 |

| | |
|---|---|
| ND = none detected | |

### EXAMPLE 10

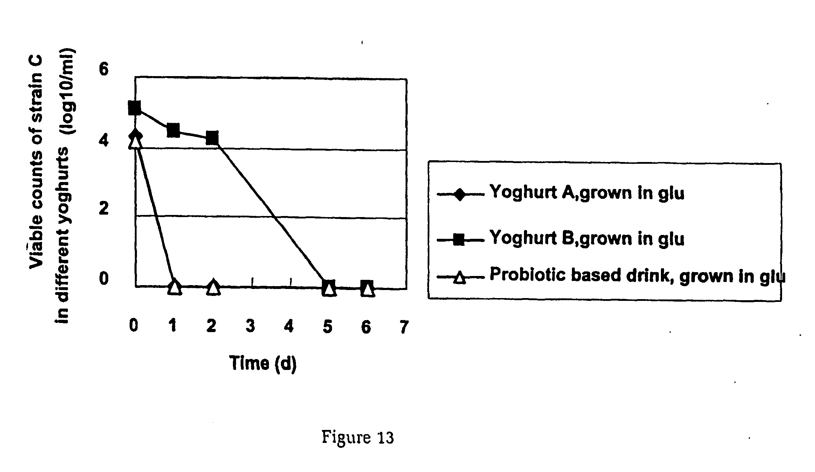

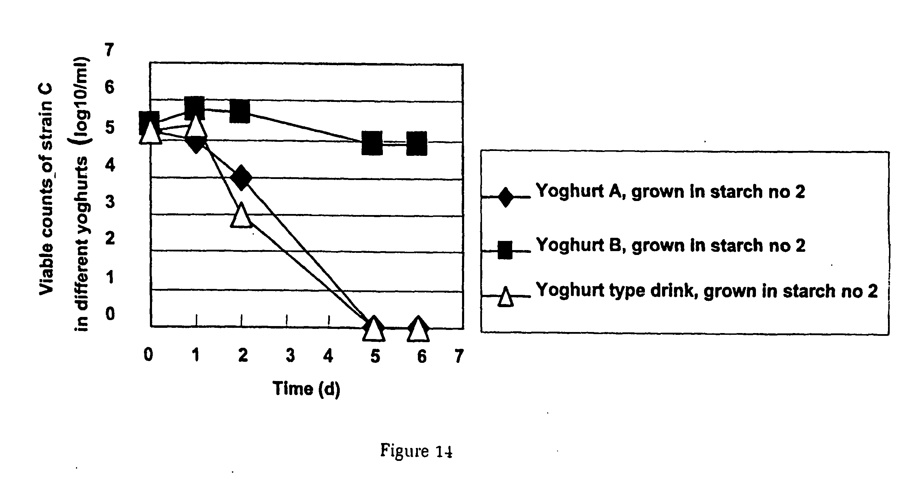

It was shown that there is synergy between food ingredients such as polysaccharides and the enhanced effect on survival of microorganisms when grown in the presence of resistant starch and/or additional resistant starch is included with the microbes. This can be demonstrated by the growth of *Bifidobacterium* strain C in the presence of glucose (0.5%), or Starch 1 (0.25%) + glucose (0.25%), or Starch 2 (0.5%), or Starch 2 (0.25%) + glucose (0.25%). The bifidobacterium cells were collected after 40 hours of anaerobic growth and the samples ere divided into two. One portion was used directly and the other portion was mixed with 2.5% Starch 1 for 1 hour. The cells alone or the cells plus starch granules were then collected by centrifuging and resuspended in a yoghurt type fermented milks which were then stored at room temperature and sampled at 0, 1, 2, 5, and 6 days for viable bifidobacterium testing. As can be seen in Figures 13 and 14, there was a demonstrable synergy between the presence of polysaccharide in yoghurt B and the growth in the presence of resistant starch compared to glucose.

### EXAMPLE 11

It was established that bacterial cells attached to a surface survive stress conditions better than unattached cells. In this example it was found that cells of bifidobacterium grown in resistant Starch 1 adhered two-fold better to Starch 1 granules than was noted for glucose grown cells. Cells of *Bifidobocterium* strain C were grown in PYG for 24 hours and after harvesting by centrifugation were resuspended in a solution of 2.5% Starch 1 at pH 7.0 or at pH 2.5. After one hour incubation, the adhesion to the starch granules was assessed and then the pH was altered from 7.0 to 2.5 and also from 2.5 to 7.0. As can be seen in Table 5, cells of strain C adhered well at pH 7.0 but not at pH 2.5 and when the pH was changed from 7.0 to 2.5, the cells remained attached while there was an increase in adhesion when the pH was raised from pH 2.5 to pH 7.0.

**Table 5. The effect of pH on adhesion of Bifidobacterium strain C to Starch 1**

| Measurement at 1h | | Measurement at 2h | |
|---|---|---|---|
| pH | Adhesion (%) | pH | Adhesion (%) |
| 7.0 | 88.8 | 7.0 | 86.7 |
| 2.5 | 18.3 | 2.5 | 13.4 |
| 7.0 | 88.1 | pH change to 2.5 | 75.5 |
| 2.5 | 19.51 | pH changes to 6.8 | 72.8 |

### USES

(i) Specific embodiments of the invention can be applied to situations for which probiotic microbes may be used, including use as prophylactic and therapeutic agents as well as in food and feed compositions for the benefit of the host
(ii) Specific embodiments of the invention can be applied to situations for which probiotic microbes may be used for non-digestive tract applications like the nasal and vaginal tracts.
(iii) Specific embodiments of the invention can be applied to situations relating to biocontrol and bioremediation.
(iv) The probiotic microorganisms can be grown in the starch-based medium and used directly or combined with additional starch after growth. These probiotic suspensions may be used directly or after freezing and/or drying in the absence or presence of further additives.
(v) The probiotic microorganisms described in (ii) above, can be added to foods and feeds either during or at the end of production.
(vi). In addition to the foods and feeds described in (iii) above, the starch may also be added to the food or feed prior to or after addition of the probiotic microorganisms.
(vii) Specific embodiments of the invention can also apply to microorganisms, including starter cultures, used for production of fermented foods wherein these microorganisms are grown in starch-based media and optionally mixed with additional starch after growth, freezing and/or drying, thereby enhancing survival of the microbes. When these microbes are added as an ingredient, additional starch may be added to the food before, during or after production.

Specific embodiments of the invention cover the fact that for many different microorganisms, including probiotic bacteria such as lactic acid bacteria and bifidobacteria, the presence of resistant starch in the growth media can in solid and liquid preparations:
increase the growth and/or yield of the microorganism; and
increase the survival rate or recovery rate of the microbes in food, health foods including nutraceutical and/or functional foods, health supplements. food and food formulations designed for infants and geriatrics, pharmaceutical products, medical foods such as enteral feeding preparations, animal feeds, feeds for companion animals, aquaculture, bird feeds and supplements, sport and performance food supplements

Although the examples provided related mainly to food and probiotic products, it will be appreciated that other microorganisms useful for different applications, for example biocontrol and bioremediation, may be rendered to have increased growth/yield potential. or increased survival/recovery rate in use.

Growth and survival can be enhanced if additional resistant starch is added to the microorganisms after growth, and even when added to cells grown in the absence of resistant starch and which are subsequently mixed with starch.

Furthermore, the microorganisms grown in the presence of resistant starch are more resistant to stresses such as aeration, shear. freezing, drying, freeze drying. high temperature, low temperature, temperature fluctuations, pressure fluctuations, high pressure, low pressure, low pH, high pH, moisture, *in vivo* conditions.

The resistant starch, including RS1, RS2, RS3 and RS4 types, can be natural starches containing resistant starch and/or modifications thereof including both chemical and enzymatic modifications, and/or mixtures thereof. Examples of two different starches were used in the examples but one skilled in the art would appreciate that other forms of resistant starch would also be suitable for use in the present invention.

The above cited enhanced growth/yield and /or survival or recovery also applies to production and storage of preparations of microorganisms.

In addition, it was surprising to note a synergy with other food ingredients including disaccharides, oligosaccharides and polysaccharides when growth/yield and/or survival or recovery of microorganisms was monitored. It would be appreciated that other food ingredients such as proteins and fats may also act synergistically.

It was also noted that resistant starch grown cells adhered better to starch granules which in turn would ensure better survival or recovery. Since the resistant starch has reduced digestibility, it would be appreciated that other indigestible compounds including proteins and lipids could also provide enhanced growth/yield and/or survival or recovery of microorganisms.

The prior art has shown that the presence of resistant starch in probiotic compositions enhances survival of the probiotic microorganisms during and after consumption (AU 687253). This earlier patent by the present applicants shows data using bifidobacteria with additional starch added. In contrast, the present invention results from the unexpected discovery that resistant starch grown cells are more robust without the addition of more starch. Surprising, when the microorganisms are grown in the presence of resistant starch, and may have additional starch added to the starch grown cells, enhanced growth/yield and /or survival or recovery of the microorganism occurs. That is, microbial cells grown in the presence of resistant starch are more robust. Furthermore, the addition of resistant starch to cells grown in the absence of starch can enhance the robustness of those cells.

It will be appreciated by persons skilled in the art that numerous variations and/or modifications may be made to the the specific embodiments of the present invention. The present embodiments are, therefore, to be considered in all respects as illustrative and not restrictive.

## Claims

1. A process of preparing a microbial preparation having an increased survival/recovery rate in a product, the process comprising growing or culturing microbes in media based on, or containing, resistant starch, and harvesting the cultured microbes, the harvested microbes having increased survival/recovery rate when subsequently incorporated in a product as compared with the same microbes grown or cultured in a media without resistant starch.

2. The process according to claim 1 wherein the product is selected from the group consisting of a food, feed, nutraceutical, pharmaceutical, biocontrol, and bioremediation product.

3. The process according to claim 2 or 3 wherein the resistant starch is type RS1, RS2, RS3 or RS4.

4. The process according to claim 2 or 3 wherein the resistant starch is derived from starch selected from the group consisting of maize, rice, barley, wheat, legumes, potatoes, and bananas.

5. The process according to any preceding claim wherein the resistant starch is derived from a starch having an amylose content of at least 40% (w/w).

6. The process according to any preceding claim wherein the resistant starch is derived from maize starch.

7. The process according to claim 6 wherein the maize starch having an amylose content of at least 70% (w/w).

8. The process according to claim 6 wherein the maize starch having an amylose content of at least 80% (w/w).

9. The process according to claim 6 wherein the maize starch having an amylose content of at least 90% (w/w).

10. The process according to any preceding claim wherein the starch is chemically, physically, and/or enzymically treated or modified.

11. The process according to claim 10 wherein the chemical modification is selected from the group consisting of oxidation, cross-bonding, etherification, esterification, acidification, dextrinisation, and mixtures thereof.

12. The process according to claim 10 wherein the physical treatment is heat-moisture treatment to enhance or increase the resistant starch content of the starch.

13. The process according to claim 10 wherein the treatment is by solvent extraction to remove fats and/or minerals from the starch.

14. The process according to any preceding claim wherein the resistant starch is used in the media at a concentration of 0.01 to 10% (w/w).

15. The process according to claim 14 wherein the resistant starch is used in the media at 0.1 to 5% (w/w).

16. The process according to claim 14 wherein the resistant starch is used in the media at 1% (w/w).

17. The process according to any preceding claim wherein in use the microbes are resistant to aeration, sheer, freeze drying, freezing, drying including high, medium and low water activity, elevated temperatures, low temperatures, pressure and pressure fluctuations, low pH, high pH, bile acids, moisture, high osmolarity, low osmolarity, high salt, or combinations thereof.

18. The process according to any preceding claim wherein the microbial preparation is a probiotic, a starter culture, a biocontrol or bioremediation product.

19. The process according to claim 18 wherein the microbes are probiotic microorganisms from the genera selected from the group of consisting of *Saccharomyces, Bifidobacterium, Bacteroides, Clostridium, Fusobacterium, Propionibacterium, Streptococcus, Enterococcus, Lactococcus, Staphylococcus,_Peptostreptococcus,* and *Lactobacillus.*

20. The process according to claim 18 wherein the microbes are starter cultures selected from the group consisting of lactic acid bacteria including lactobacillus, lactococcus and streptococcus, leuconostoc, and yeasts.

21. The process according to claim 18 wherein the microbes are suitable for use in biocontrol or bioremediation being selected from the group consisting of bifidobacteria, acidophilus, fungi, *Bacillus* species, pseudomonads and Alcaligenes.

22. A microbial preparation containing microbes having an increased survival recovery/rate in product as compared with the same microbes grown or cultured in a media without resistant starch preparable by the process according to any of the preceding claims.

23. A product containing microbes having an increased survival recovery/rate as compared with the same microbes grown or cultured in a media without resistant starch, the product including a microbial preparation according to claim 22.

24. The product according to claim 23 selected from the group consisting of a food, feed, nutraceutical, pharmaceutical, biocontrol, and bioremediation product.

25. The product according to claim 24 being a food, feed, nutraceutical or pharmaceutical product selected from the group consisting of fluid-based food products, water-based fluids, cereal and plant-based food products, solid-based food products, tablets, food additives, health supplements, and pharmaceutical preparations.

26. The product according to claim 25 wherein the fluid-based food products include milk-based products where the edible ingredient is one or more milk-based ingredients including whole milk, milk solids, milk fat, cream, non-fat dried milk, any other component or derivative from milk suitable for use in milk-based products.

27. The product according to claim 25 wherein the products include snack bars, breakfast cereals, bread, confectionary, extruded food products, muesli bars, buns, biscuits, feed pellets, and coated food products.

28. The product according to claim 24 being a food product suitable to contain and deliver probiotic microorganisms.

29. The food product according to claim 28 selected from the group consisting of food stuffs, fruit beverages, water ices, confectionary, coatings or covertures, yoghurts, yoghurt drinks, unfermented drinks, flavoured milk drinks, modified milk drinks, ice-creams, and dairy desserts.

30. Use of resistant starch in microbial culture media to produce microbes which when subsequently used in a product after being harvested from the media have an increased survival rate as compared with the same microbes grown or cultured in media without resistant starch.

31. The use according to claim 30 wherein the product is selected from the group consisting of a food, feed, nutraceutical, pharmaceutical, biocontrol, and bioremediation product.

32. The use according to claim 30 or 31 wherein the resistant starch is type RS1, RS2, RS3 or RS4.

33. The use according to claim 32 wherein the resistant starch is derived from starch selected from the group consisting of maize, rice, barley, wheat, legumes, potatoes, and bananas.

34. The use according to any of claims 30-33 wherein the resistant starch is derived from a starch having an amylose content of at least 40% (w/w).

35. The use according to claim 34 wherein the resistant starch is derived from maize starch.

36. The use according to claim 35 wherein the maize starch having an amylose content of at least 70% (w/w).

37. The use according to claim 35 wherein the maize starch having an amylose content of at least 80% (w/w).

38. The use according to claim 35 wherein the maize starch having an amylose content of at least 90% (w/w).

39. The use according to any of claims 30-38 wherein the starch is chemically, physically, and/or enzymically treated or modified.

40. The use according to claim 39 wherein the chemical modification is selected from the group consisting of oxidation, cross-bonding, etherification, esterification, acidification, dextrinisation, and mixtures thereof.

41. The use according to claim 39 wherein the physical treatment is heat-moisture treatment to enhance or increase the resistant starch content of the starch.

42. The use according to claim 39 wherein the treatment is by solvent extraction to remove fats and/or minerals from the starch.

## Patentansprüche

1. Verfahren zur Herstellung einer Mikrobenzubereitung mit erhöhter Überlebens-/Rückgewinnungsrate in einem Produkt, wobei das Verfahren Züchten oder Kultivieren von Mikroben in einem Medium, das auf resistenter Stärke basiert oder diese enthält, und Ernten der kultivierten Mikroben umfasst, wobei die geernteten Mikroben verglichen mit den gleichen Mikroben, die in einem Medium ohne resistente Stärke gezüchtet wurden, eine erhöhte Überlebens-/Rückgewinnungsrate aufweisen, wenn sie anschließend in ein Produkt eingebracht werden.

2. Verfahren nach Anspruch 1, wobei das Produkt aus der Gruppe ausgewählt ist, bestehend aus einem Nahrungsmittel-, Futter-, nutrazeutischen, pharmazeutischen, Biokontroll- und Bioremediationsprodukt.

3. Verfahren nach Anspruch 2 oder 3, wobei die resistente Stärke vom Typ RS1, RS2, RS3 oder RS4 ist.

4. Verfahren nach Anspruch 2 oder 3, wobei die resistente Stärke von Stärke stammt, die aus der Gruppe, bestehend aus Mais, Reis, Gerste, Weizen, Hülsenfrüchten, Kartoffeln und Bananen, ausgewählt ist.

5. Verfahren nach einem vorhergehenden Anspruch, wobei die resistente Stärke von einer Stärke mit einem Amylosegehalt von mindestens 40% (w/w) stammt.

6. Verfahren nach einem vorhergehenden Anspruch, wobei die resistente Stärke von Maisstärke stammt.

7. Verfahren nach Anspruch 6, wobei die Maisstärke einen Amylosegehalt von mindestens 70% (w/w) besitzt.

8. Verfahren nach Anspruch 6, wobei die Maisstärke einen Amylosegehalt von mindestens 80% (w/w) besitzt.

9. Verfahren nach Anspruch 6, wobei die Maisstärke einen Amylosegehalt von mindestens 90% (w/w) besitzt.

10. Verfahren nach einem vorhergehenden Anspruch, wobei die Stärke chemisch, physikalisch und/oder enzymatisch behandelt oder modifiziert ist.

11. Verfahren nach Anspruch 10, wobei die chemische Modifikation aus der Gruppe ausgewählt ist, bestehend aus Oxidation, Quervernetzung, Veretherung, Veresterung, Ansäuerung, Dextrinisierung und Gemischen davon.

12. Verfahren nach Anspruch 10, wobei die physikalische Behandlung Wärme-Feuchtigkeits-Behandlung ist, um den Gehalt der Stärke an resistenter Stärker zu steigern oder zu erhöhen.

13. Verfahren nach Anspruch 10, wobei die Behandlung durch Lösungsmittelextraktion zur Entfernung von Fetten und/oder Mineralien aus der Stärke erfolgt.

14. Verfahren nach einem vorhergehenden Anspruch, wobei die resistente Stärke in dem Medium in einer Konzentration von 0,01 bis 10% (w/w) verwendet wird.

15. Verfahren nach Anspruch 14, wobei die resistente Stärke in dem Medium zu 0,1 bis 5% (w/w) verwendet wird.

16. Verfahren nach Anspruch 14, wobei die resistente Stärke in dem Medium zu 1% (w/w) verwendet wird.

17. Verfahren nach einem vorhergehenden Anspruch, wobei bei Verwendung die Mikroben gegenüber Belüftung, Scherung, Gefriertrocknen, Gefrieren, Trocknen einschließlich hoher, mittlerer und niedriger Wasseraktivität, erhöhten Temperaturen, niedrigen Temperaturen, Druck und Druckschwankungen, niedrigem pH, hohem pH, Gallensäuren, Feuchtigkeit, hoher Osmolarität, niedriger Osmolarität, Hochsalz oder Kombinationen davon resistent sind.

18. Verfahren nach einem vorhergehenden Anspruch, wobei die Mikrobenzubereitung ein probiotisches, ein Starterkultur-, ein Biokontroll- oder ein Bioremediationsprodukt ist.

19. Verfahren nach Anspruch 18, wobei die Mikroben probiotische Mikroorganismen aus den Gattungen sind, die aus der Gruppe, bestehend aus *Saccharomyces, Bifidobacterium, Bacteroides, Clostridium, Fusobacterium, Propionibacterium, Streptococcus, Enterococcus, Lactococcus, Staphylococcus, Peptostreptococcus* und *Lactobacillus,* ausgewählt sind.

20. Verfahren nach Anspruch 18, wobei die Mikroben Starterkulturen sind, die aus der Gruppe, bestehend aus Milchsäurebakterien, einschließlich Lactobacillus, Lactococcus und Streptococcus, Leuconostoc und Hefen, ausgewählt sind.

21. Verfahren nach Anspruch 18, wobei die Mikroben für die Verwendung zur Biokontrolle oder Bioremediation geeignet sind und aus der Gruppe, bestehend aus Bifidobakterien, Acidophilus, Pilzen, Bacillus-Spezies, Pseudomonaden und Alkaligenes, ausgewählt sind.

22. Mikrobenzubereitung, enthaltend Mikroben mit erhöhter Überlebens-/Rückgewinnungsrate verglichen mit den gleichen Mikroben, die in einem Medium ohne resistente Stärke gezüchtet oder kultiviert wurden, die durch das Verfahren nach einem der vorhergehenden Ansprüche erhältlich sind.

23. Produkt, enthaltend Mikroben mit erhöhter Überlebens-/Rückgewinnungsrate verglichen mit den gleichen Mikroben, die in einem Medium ohne resistente Stärke gezüchtet oder kultiviert wurden, wobei das Produkt eine Mikrobenzubereitung nach Anspruch 22 enthält.

24. Produkt nach Anspruch 23, ausgewählt aus der Gruppe, bestehend aus einem Nahrungsmittel-, Futter-, nutrazeutischen, pharmazeutischen, Biokontroll- und Bioremediationsprodukt.

25. Produkt nach Anspruch 24, das ein Nahrungsmittel-, Futter-, nutrazeutisches oder pharmazeutisches Produkt ist, ausgewählt aus der Gruppe, bestehend aus Nahrungsmittelprodukten auf Flüssigkeitsbasis, Flüssigkeiten auf Wasserbasis, Nahrungsmittelprodukten auf Getreide- und Pflanzenbasis, Nahrungsmittelprodukten auf Feststoffbasis, Tabletten, Nahrungsmittelzusatzstoffen, Gesundheitszusätzen und pharmazeutischen Zubereitungen.

26. Produkt nach Anspruch 25, wobei die Nahrungsmittelprodukte auf Flüssigkeitsbasis Produkte auf Milchbasis umfassen, wobei der essbare Inhaltsstoff ein oder mehrere Inhaltsstoffe auf Milchbasis ist, einschließlich Vollmilch, Milchfeststoffen, Milchfett, Sahne, fettfreier Trockenmilch, jeder anderen Komponente oder jedes anderen Derivats von Milch, die/das sich zur Verwendung in Produkten auf Milchbasis eignet.

27. Produkt nach Anspruch 25, wobei die Nahrungsmittelprodukte auf Feststoffbasis Snackriegel, Frühstücksgetreide, Brot, Süßwaren, extrudierte Nahrungsmittelprodukte, Müsliriegel, Brötchen, Kekse, Futterpellets und überzogene Nahrungsmittelprodukte enthalten.

28. Produkt nach Anspruch 24, das ein Nahrungsmittelprodukt ist, das dazu geeignet ist, probiotische Mikroorganismen zu enthalten und zuzuführen.

29. Nahrungsmittelprodukt nach Anspruch 28, das aus der Gruppe ausgewählt ist, bestehend aus Nährmitteln, Fruchtgetränken, Wassereis, Süßwaren, Überzügen oder Kuvertüren, Joghurts, Joghurtgetränken, unfermentierten Getränken, aromatisierten Milchgetränken, modifizierten Milchgetränken, Eiscremes und Milchdesserts.

30. Verwendung von resistenter Stärke in Mikrobenkulturmedium zur Herstellung von Mikroben, die, wenn sie anschließend in einem Produkt verwendet werden, nachdem sie aus dem Medium geerntet wurden, eine erhöhte Überlebensrate verglichen mit den gleichen Mikroben, die in Medium ohne resistente Stärke gezüchtet oder kultiviert wurden, besitzen.

31. Verwendung nach Anspruch 30, wobei das Produkt aus der Gruppe, bestehend aus einem Nahrungsmittel-, Futter-, nutrazeutischen, pharmazeutischen, Biokontroll- und Bioremediationsprodukt, ausgewählt ist.

32. Verwendung nach Anspruch 30 oder 31, wobei die resistente Stärke vom Typ RS1, RS2, RS3 oder RS4 ist.

33. Verwendung nach Anspruch 32, wobei die resistente Stärke von Stärke stammt, die aus der Gruppe, bestehend aus Mais, Reis, Gerste, Weizen, Hülsenfrüchten, Kartoffeln und Bananen, ausgewählt ist.

34. Verwendung nach einem der Ansprüche 30-33, wobei die resistente Stärke von einer Stärke mit einem Amylosegehalt von mindestens 40% (w/w) stammt.

35. Verwendung nach Anspruch 34, wobei die resistente Stärke von Maisstärke stammt.

36. Verwendung nach Anspruch 35, wobei die Maisstärke einen Amylosegehalt von mindestens 70% (w/w) besitzt.

37. Verwendung nach Anspruch 35, wobei die Maisstärke einen Amylosegehalt von mindestens 80% (w/w) besitzt.

38. Verwendung nach Anspruch 35, wobei die Maisstärke einen Amylosegehalt von mindestens 90% (w/w) besitzt.

39. Verwendung nach einem der Ansprüche 30-38, wobei die Stärke chemisch, physikalisch und/oder enzymatisch behandelt oder modifiziert ist.

40. Verwendung nach Anspruch 39, wobei die chemische Modifikation aus der Gruppe ausgewählt ist, bestehend aus Oxidation, Quervernetzung, Veretherung, Veresterung, Ansäuerung, Dextrinisierung und Gemischen davon.

41. Verwendung nach Anspruch 39, wobei die physikalische Behandlung Wärme-Feuchtigkeits-Behandlung ist, um den Gehalt der Stärke an resistenter Stärke zu steigern oder zu erhöhen.

42. Verwendung nach Anspruch 39, wobei die Behandlung durch Lösungsmittelextraktion zur Entfernung von Fetten und/oder Mineralien aus der Stärke erfolgt.

## Revendications

1. Procédé de préparation d'une préparation microbienne présentant un taux accru de survie/récupération dans un produit, le procédé comprenant les étapes consistant à faire pousser ou mettre en culture des microbes dans un milieu à base de, ou contenant de l'amidon résistant et récolter les microbes cultivés, les microbes récoltés présentant un taux accru de survie/récupération lorsqu'ils sont incorporés par la suite dans un produit par comparaison avec les mêmes microbes ayant poussé ou cultivés dans un milieu sans amidon résistant.

2. Procédé selon la revendication 1, **caractérisé en ce que** le produit est choisi dans le groupe constitué d'un produit alimentaire, d'un produit alimentaire pour animaux, d'un produit nutraceutique, d'un produit pharmaceutique, d'un produit de biocontrôle et d'un produit de bioremédiation.

3. Procédé selon la revendication 2 ou 3, **caractérisé en ce que** l'amidon résistant est du type AR1, AR2, AR3 ou AR4.

4. Procédé selon la revendication 2 ou 3, **caractérisé en ce que** l'amidon résistant est dérivé d'un amidon choisi dans le groupe constitué du maïs, du riz, de l'orge, du blé, des légumineuses, des pommes de terre et des bananes.

5. Procédé selon l'une quelconque des revendications précédentes, **caractérisé en ce que** l'amidon résistant est dérivé d'un amidon ayant une teneur en amylose d'au moins 40 % (p/p).

6. Procédé selon l'une quelconque des revendications précédentes, **caractérisé en ce que** l'amidon résistant est dérivé de l'amidon de maïs.

7. Procédé selon la revendication 6, **caractérisé en ce que** l'amidon de maïs a une teneur en amylose d'au moins 70 % (p/p).

8. Procédé selon la revendication 6, **caractérisé en ce que** l'amidon de maïs a une teneur en amylose d'au moins 80 % (p/p).

9. Procédé selon la revendication 6, **caractérisé en ce que** l'amidon de maïs a une teneur en amylose d'au moins 90 % (p/p).

10. Procédé selon l'une quelconque des revendications précédentes, **caractérisé en ce que** l'amidon est traité ou modifié chimiquement, physiquement et/ou enzymatiquement.

11. Procédé selon la revendication 10, **caractérisé en ce que** la modification chimique est choisie dans le groupe constitué d'une oxydation, d'une réticulation, d'une éthérification, d'une estérification, d'une acidification, d'une dextrinisation et de mélanges de celles-ci.

12. Procédé selon la revendication 10, **caractérisé en ce que** le traitement physique est un traitement hydrothermique destiné à amplifier ou à accroître la teneur en amidon résistant de l'amidon.

13. Procédé selon la revendication 10, **caractérisé en ce que** le traitement se fait par extraction par solvant afin d'éliminer les graisses et/ou les minéraux de l'amidon.

14. Procédé selon l'une quelconque des revendications précédentes, **caractérisé en ce que** l'amidon résistant est utilisé dans le milieu à une concentration de 0,01 à 10 % (p/p).

15. Procédé selon la revendication 14, **caractérisé en ce que** l'amidon résistant est utilisé dans le milieu à de 0,1 à 5 % (p/p).

16. Procédé selon la revendication 14, **caractérisé en ce que** l'amidon résistant est utilisé dans le milieu à 1 % (p/p).

17. Procédé selon l'une quelconque des revendications précédentes, **caractérisé en ce que**, en utilisation, les microbes sont résistants à l'aération, au cisaillement, à la lyophilisation, à la congélation, à la dessication, y compris à une activité de l'eau élevée, moyenne ou faible, aux températures élevées, aux basses températures, à la pression et aux fluctuationx de pression, aux pH faibles, aux pH élevés, aux acides biliaires, à l'humidité, à une forte osmolarité, à une faible osmolarité, aux concentrations en sel élevées ou à des combinaisons de ceux-ci.

18. Procédé selon l'une quelconque des revendications précédentes, **caractérisé en ce que** la préparation microbienne est un probiotique, un levain, un produit de biocontrôle ou de bioremédiation.

19. Procédé selon la revendication 18, **caractérisé en ce que** les microbes sont des microorganismes probiotiques des genres choisis dans le groupe constitué de *Saccharomyces, Bifidobacterium, Bacteroides, Clostridium, Fusobacterium, Propionibacterium, Streptococcus, Enterococcus, Lactococcus, Staphylococcus, Peptostreptococcus* et *Lactobacillus.*

20. Procédé selon la revendication 18, **caractérisé en ce que** les microbes sont des levains choisis dans le groupe constitué des bactéries lactiques notamment lactobacillus, lactococcus et streptococcus, leuconostoc et des levures.

21. Procédé selon la revendication 18, **caractérisé en ce que** les microbes sont appropriés pour une utilisation dans le biocontrôle et la bioremédiation, étant choisis dans le groupe constitué des bifidobactéries, d'acidophilus, des champignons, des espèces de *Bacillus,* des pseudomonadacées et des Alcaligènes.

22. Préparation microbienne contenant des microbes présentant un taux accru de survie/récupération dans un produit par comparaison avec les mêmes microbes ayant poussé ou cultivés dans un milieu sans amidon résistant, pouvant être préparée par le procédé selon l'une quelconque des revendications précédentes.

23. Produit contenant des microbes présentant un taux de accru survie/récupération par comparaison avec les mêmes microbes ayant poussé ou cultivés dans un milieu sans amidon résistant, le produit comprenant une préparation microbienne selon la revendication 22.

24. Produit selon la revendication 23, choisi dans le groupe constitué d'un produit alimentaire, d'un produit alimentaire pour animaux, d'un produit nutraceutique, d'un produit pharmaceutique, d'un produit de biocontrôle et d'un produit de bioremédiation.

25. Produit selon la revendication 24, étant un produit alimentaire, un produit alimentaire pour animaux, un produit nutraceutique ou un produit pharmaceutique choisi dans le groupe constitué des produits alimentaires à base de fluides, des fluides à base d'eau, des produits alimentaires à base de céréales et à base de plantes, des produits alimentaires à base de solides, des comprimés, des additifs alimentaires, des compléments diététiques et des préparations pharmaceutiques.

26. Produit selon la revendication 25, **caractérisé en ce que** les produits alimentaires à base de fluides comprennent des produits à base de lait où l'ingrédient comestible est un ou plusieurs ingrédients à base de lait notamment du lait entier, des matières sèches du lait, de la matière grasse du lait, de la crème, du lait sec écrémé, tout autre composant ou dérivé du lait approprié pour une utilisation dans des produits à base de lait.

27. Produit selon la revendication 25, **caractérisé en ce que** les produits alimentaires à base de solides comprennent des barres en-cas, des céréales pour le petit déjeuner, du pain, des pâtisseries, des produits alimentaires extrudés, des barres de muesli, des brioches, des biscuits, des granulés d'aliments pour animaux et des produits alimentaires enrobés.

28. Produit selon la revendication 24, étant un produit alimentaire approprié pour contenir et délivrer des microorganismes probiotiques.

29. Produit alimentaire selon la revendication 28, choisi dans le groupe constitué des denrées alimentaires, des boissons aux fruits, des glaces à l'eau, des pâtisseries, des enrobages ou nappages, des yaourts, des boissons au yaourt, des boissons non fermentées, des boissons au lait aromatisé, des boissons au lait modifié, des crèmes glacées et des desserts à base de lait.

30. Utilisation d'amidon résistant dans un milieu de culture microbien, pour produire des microbes qui, lorsqu'ils sont utilisés par la suite dans un produit après avoir été récoltés à partir du milieu, présentent un taux de survie accru par comparaison avec les mêmes microbes ayant poussé ou cultivés dans un milieu sans amidon résistant.

31. Utilisation selon la revendication 30, **caractérisée en ce que** le produit est choisi dans le groupe constitué d'un produit alimentaire, d'un produit alimentaire pour animaux, d'un produit nutraceutique, d'un produit pharmaceutique, d'un produit de biocontrôle et d'un produit de bioremédiation.

32. Utilisation selon la revendication 30 ou 31, **caractérisée en ce que** l'amidon résistant est du type AR1, AR2, AR3 ou AR4.

33. Utilisation selon la revendication 32, **caractérisée en ce que** l'amidon résistant est dérivé d'un amidon choisi dans le groupe constitué du maïs, du riz, de l'orge, du blé, des légumineuses, des pommes de terre et des bananes.

34. Utilisation selon l'une quelconque des revendications 30-33, **caractérisée en ce que** l'amidon résistant est dérivé d'un amidon ayant une teneur en amylose d'au moins 40 % (p/p).

35. Utilisation selon la revendication 34, **caractérisée en ce que** l'amidon résistant est dérivé de l'amidon de maïs.

36. Utilisation selon la revendication 35, **caractérisée en ce que** l'amidon de maïs a une teneur en amylose d'au moins 70 % (p/p).

37. Utilisation selon la revendication 35, **caractérisée en ce que** l'amidon de maïs a une teneur en amylose d'au moins 80 % (p/p).

38. Utilisation selon la revendication 35, **caractérisée en ce que** l'amidon de maïs a une teneur en amylose d'au moins 90 % (p/p).

39. Utilisation selon l'une quelconque des revendications 30-38, **caractérisée en ce que** l'amidon est traité ou modifié chimiquement, physiquement et/ou enzymatiquement.

40. Utilisation selon la revendication 39, **caractérisée en ce que** la modification chimique est choisie dans le groupe constitué d'une oxydation, d'une réticulation, d'une éthérification, d'une estérification, d'une acidification, d'une dextrinisation et de mélanges de celles-ci.

41. Utilisation selon la revendication 39, **caractérisée en ce que** le traitement physique est un traitement hydrothermique destiné à amplifier ou à accroître la teneur en amidon résistant de l'amidon.

42. Utilisation selon la revendication 39, **caractérisée en ce que** le traitement se fait par extraction par solvant afin d'éliminer les graisses et/ou les minéraux de l'amidon.
